Europäisches Patentamt

European Patent Office

Office européen des brevets

(19)

(11) Veröffentlichungsnummer: **0 008 115**
**B1**

(12) **EUROPÄISCHE PATENTSCHRIFT**

(45) Veröffentlichungstag der Patentschrift: **16.02.83**

(21) Anmeldenummer: **79102860.8**

(22) Anmeldetag: **08.08.79**

(51) Int. Cl.³: **C 07 D 303/16,**
**C 07 D 301/14**

(54) **Verfahren zur Herstellung von Fumarsäureglycidylestern.**

(30) Priorität: **16.08.78 DE 2835882**

(43) Veröffentlichungstag der Anmeldung:
**20.02.80 Patentblatt 80/4**

(45) Bekanntmachung des Hinweises auf die Patenterteilung:
**16.02.83 Patentblatt 83/7**

(84) Benannte Vertragsstaaten:
**CH DE FR GB IT NL**

(56) Entgegenhaltungen:
**DE - A - 1 942 557**
**DE - A - 2 602 776**
**DE - B - 1 082 263**
**DE - B - 1 083 797**
**FR - A - 1 269 628**

(73) Patentinhaber: **BAYER AG**
**Zentralbereich Patente, Marken und Lizenzen**
**D-5090 Leverkusen 1, Bayerwerk (DE)**

(72) Erfinder: **Rauleder, Gebhard, Dr.**
**Mozartstrasse 20**
**D-5657 Haan (DE)**
Erfinder: **Waldmann, Helmut, Dr.**
**Carl-Rumpff-Strasse 59**
**D-5090 Leverkusen 1 (DE)**
Erfinder: **Bottenbruch, Ludwig, Dr.**
**Wöhlerstrasse 5**
**D-4150 Krefeld 1 (DE)**
Erfinder: **Prater, Klaus, Dr.**
**Bodelschwinghstrasse 22**
**D-4150 Krefeld 1 (DE)**
Erfinder: **Seifert, Hermann, Dr.**
**Ruwergasse 4**
**D-5000 Köln 80 (DE)**
Erfinder: **Swodenk, Wolfgang, Dr.**
**Auf dem Broich 5**
**D-5068 Odenthal (DE)**

# Verfahren zur Herstellung von Fumarsäureglycidylestern

Die vorliegende Erfindung betrifft eine Verfahren zur Herstellung von Fumarsäureglycidylestern aus Fumarsäurediallylester.

Fumarsäureglycidylester können als Ausgangsprodukte zur Herstellung von Gießharzen, in Pulverbeschichtungsmitteln, als Stabilisator und als Ausgangsprodukt für die Herstellung von Lacken, Kunststoffen und Klebstoffen Verwendung finden (s. z.B. US—A—3 155 638 und JP—A—75/59 429). Ein technischer Einsatz dieser Produkte ist in größerem Maße bisher nicht erfolgt, da noch kein befriedigendes Verfahren zur ihrer Herstellung ver Verfügung gestanden hat.

Es ist bekannt, daß man Glycidylester aus Carbonsäuren und Salzen von Carbonsäuren durch Umsetzung mit Epihalogenhydrin herstellen kann (s. z.B. DE—B—1 165 030, DE—A—1 643 777, US—A—2 448 602 und DE—A—2 023 148). Ein grundsätzlicher Nachteil dieser Verfahren besteht darin, daß salzhaltige umweltbelastende Abwässer gebildet werden.

In der GB—A—735 001 wird die Herstellung Glycidylestern durch Umsetzen von Glycid mit Polycarbonsäurechloriden beschrieben. Dieses Verfahren hat den großen Nachteil, daß die Ausgangsmaterialien instabil und sehr reaktionsfähig sind, so daß bei der Herstellung, der Lagerung und der Verwendung dieser Verbindungen äußerste Vorsicht erforderlich ist (vgl. DE—A—2 032 148, s. 1, Z. 6).

Eine Möglichkeit, Olefine in Epoxide umzuwandeln unter Umgehung dieser Nachteile, besteht in der Anwendung der "Prileschajew-Reaktion" (vgl. N. Prileschajew, Ber dtsch. chem. Ges. *42*, 4811, (1909)). Bei dieser Reaktion handelt es sich um einem elektrophilen Angriff einer Percarbonsäure auf ein Olefin (vgl. K. D. Bingham,, G. D. Meakins, G. H. Whitham, Chem. Commun. 1966, S. 445 und 446). Aus diesem Grunde nimmit die Reaktivität des Olefins mit fallender Nucleophilie der Doppelbindung ab. Deshalb erschweren elektronegative Substituenten die Epoxidation (vgl. S. N. Lewis in R. L. Augustin, "Oxidation" Vol. I, S. 227, Z. 9—13, Marcel Dekker, New York (1969)).

Elektronenziehende Gruppierungen wie z.B. Carboxyl, Carbonyl usw. vermindern die Reaktionsgeschwindigkeit enorm (vgl. H. Batzer und E. Nikles, Chimia, Vol. 16, Seite 62 (1962)). Insbesondere lassen sich daher Allylester nicht ohne weiteres mit Percarbonsäuren epoxidieren (vgl. DE—A—2 023 148). Infolge der geringen Reaktionsfähigkeit ihrer Doppelbindung sind hohe Temperaturen und lange Reaktionszeiten erforderlich, was zur Bildung von unerwünschten Nebenprodukten, wie Dihydroxy- und Hydroxyacyloxy-Derivaten der Ausgangsprodukte, Anlaß gibt (vgl. S. N. Lewis in R. L. Augustin. "Oxidation" Vol. 1, S. 233, Z. 6—11, Marcel Dekker, New York (1969)).

In der US—A—3 155 638, Beispiel 13, wird nach der Umsetzung von Fumarsäurediallylester mit Phthalsäureanhydrid und Wasserstoffperoxid die Phthalsäure abfiltriert. Der im Filtrat verbleibende Rest muß durch wiederholtes Waschen mit Natronlauge und Wasser rückgewonnen werden und dann anschließend müssen zur Wiedergewinnung der in den wäßrigen Phasen enthaltenen Phthalsäure diese wieder angesäuert werden, was zur Bildung unerwünschter Salzabfälle führt. Bei diesem Verfahren wird nur Allylglycidylfumarat und dieses nur in einer Ausbeute von 34% d.Th. erhalten.

In der US—A—2 761 870 wird die Epoxidierung von Fumarsäuredicrotylester mit 45% iger Peressigsäure beschrieben. Die angegebenen Reaktionsbedingungen — das Reaktionsgemisch muß 2 Tage bei 0 bis 25°C stehen — sind für eine technische Anwendung äußerst aufwendig. Nach der Reaktion wird die Essigsäure durch Waschen mit 20% iger Natronlauge aus dem Reaktionsgemisch entfernt. Will man die Essigsäure wiedergewinnen, muß die wäßrige Phase wieder angesäuert werden, was zur Bildung umweltbelastender Salzabfälle führt.

Aus der DE—B—1 083 797 ist ein Verfahren zur Herstellung von Epoxiden durch Umsetzung von Monoolefinen mit 7 bis 12 C-Atomen mit Peressigsäure bekannt, bei den man das Reaktionsgemisch in Gegenwart eines Überschusses an Olefin destilliert, der ausreicht, um die entstandene Essigsäure als azeotropes Gemisch zu entfernen. Diese Destillation wird bei Temperaturen unter 100°C durchgeführt und man kann in Gegenwart eines über dem Siedepunkt des Epoxids siedenden Treibmittels arbeiten. Das erfindungsgemäße Verfahren unterscheidet sich von diesem Verfahren wesentlich. So entstehen erfindungsgemäß höher als Essigsäure siedende Carbonsäuren, von denen nicht bekannt ist, ob sie mit Fumarsäurediallylester Azeotrope bilden können. Solche Azeotrope hätten in jedem Fall aber wesentlich höhere Siedepunkte als die gemäß der DE—B—1 083 797 auftretenden. Außerdem kann der erfindungsgemäß einzusetzende Allylester nicht nicht im Überschuß eingesetzt werden weil sonst die Bildung von Diglycidylester nicht mehr möglich ist. Der erfindungsgemäß zuzufügende Zusatzstoff soll zwischen der entstehenden Carbonsäure und dem nächst höher siedenden Bestandteil sieden, nicht oberhalb des gebildeten Epoxids. Erfindungsgemäß kann die Destillation nicht auf Temperaturen unter 100°C begrenzt werden, in der DE—B—1 083 797 wird dagegen ausgesagt, daß bereits über 50°C die gebildete Carbonsäure Epoxide aufspalten kann, und erfindungsgemäß können Bis-Epoxide vorliegen, die erhöhte Angriffsmöglichkeiten haben im Vergleich zu den Monoepoxiden, die gemäß der DE—B—1 083 797 erhalten werden. Schlie-

ßlich geht aus dieser Druckschrift nicht hervor, daß das dort beschriebene Verfahren zur Epoxidation von Diallylestern geeignet ist, die bekanntermaßen schwierig zu epoxidieren sind. Aus allen diesen Gründen kann das Verfahren der DE—B—1 083 797 das Erfindungsgemäße Verfahren nicht nahelegen.

Es wurde nun ein Verfahren zur Herstellung von Fuamrsäureglycidylestern gefunden, das dadurch gekennzeichnet ist, daß man Fumarsäurediallylester mit einer 3 bis 4 Kohlenstoffatome enthaltenden Percarbonsäure in Gegenwart von organischen Lösungsmitteln bei einer Temperatur von 30 bis 100°C umsetzt und das erhaltene Reaktionsgemisch durch Destillation aufarbeitet, wobei man die aus der Percarbonsäure entstandene Carbonsäure abtrennt, indem man dem Reaktionsgemisch einen Zusatzstoff zufügt, der zwischen der aus der Percarbonsäure entstehenden Carbonsäure und dem nächst höher siedenden Bestandteil des Reaktionsgemisches siedet und die Carbonsäure zusammen mit dem Lösungsmittel abdestilliert.

Nach dem erfindungsgemäßen Verfahren einsetzbarer Fumarsäurediallylester kann in an sich bekannter Weise erhalten werden, beispielsweise durch Veresterung von Fumarsäure mit allylalkohol.

Als organische Lösungsmittel können im erfindungsgemäßen Verfahren die verschiedensten unsubstituierten und substituierten Kohlenwasserstoffe verwendet werden, die unter Reaktionsbedingungen flüssig sind und unerwünschte Nebenreaktionen nicht oder nur in ganz untergeordnetem Maße eingehen. Als Kohlenwasserstoffe können z.B. verwendet werden aliphatische und cycloaliphatische Kohlenwasserstoffe wie Hexan, Heptan, Octan, 2 - Äthyl-hexan, Decan, Dodecan, Cyclohexan, Methylcyclopentan und Petroläther, weiterhin aromatische Kohlenwasserstoffe wie Benzol, Nitrobenzol, Toluol, Athylbenzol, Cumol, Diisopropylbenzol, Xylol und Chlorbenzol, weiterhin sauerstoffhaltige Kohlenwasserstoffe wie Diäthyläther, Diisopropyläther, Dibutyläther, Tetrahydrofuran, Dioxan, Essigsäureäthylester, Essigsäuremethylester, Essigsäurepropylester, Essigsäurebutylester, Propionsäuremethylester, Propionsäureäthylester, Propionsäurepropylester, Buttersäuremethylester, Buttersäureäthylester, Buttersäurepropylester Buttersäurebutylester, Benzoesäuremethylester und Benzoesäureäthylester, weiterhin chlorierte Kohlenwasserstoffe wie Methylenchlorid, Chloroform, Tetrachlorkohlenstoff, 1 - Chloräthan, 1,2 - Dichloräthan, 1,1 - Dichloräthan, 1,1,2,2 - Tetrachloräthan, 1 - Chlorpropan, 2 - Chlorpropan, 1,2 - Dichlorpropan, 1,3 - Dichlorpropan, 2,3 - Dichlorpropan, 1,2,3 - Trichlorpropan, 1,1,2,3 - Tetrachlorpropan, Butylchlorid, 1,2 - Dichlorbutan, 1,4 - Dichlorbutan, 2,3 - Dichlorbutan, 1,3 - Dichlorbutan, 1,2,3,4 - Tetrachlorbutan, tert. - Butylchlorid, Amylchlorid, 1,2 - Dichlorpentan, 1,5 - Dichlorpentan, 1,2,3,4 - Tetrachlorpentan, Cyclopentylchlorid, 1,2 - Dichlorcyclopentylchlorid, Hexylchlorid, 1,2 - Dichlorhexan, 1,6 - Dichlorhexan, 1,2,3,4 - Tetrachlorhexan, 1,2,5,6 - Tetrachlorhexan, Cyclohexylchlorid, Chlorbenzol, Heptylchlorid, 1,2 - Dichlorheptan, 1,2,3,4 - Tetrachlorheptan, Cycloheptylchlorid, Octylchlorid, 1,2 - Dichloroctan, 1,2,3,4 - Tetrachloroctan und Cyclooctylchlorid.

Bevorzute Lösungsmittel sind von der chlorierten Kohlenwasserstoffen Methylenchlorid, Chloroform, Tetrachlorkohlenstoff und 1,2-Dichlorpropan, von den aromatischen Kohlenwasserstoffen Benzol, Nitrobenzol, Toluol und Chlorbenzol, von den aliphatischen und cycloaliphatischen Kohlenwasserstoffen 2 - Äthy-hexan, Cyclohexan und Methylcyclopentan und von den sauerstoffhaltigen Kohlenwasserstoffen Tetrahydrofuran, Propionsäureäthylester und Benzoesäureäthylester.

Besonders bevorzugte Losungsmittel sind von der chlorierten Kohlenwasserstoffen 1,2 - Dichlorpropan und Tetrachlorkohlenstoff, von dem aromatischen Kohlenwasserstoffen Benzol und Chlorbenzol, von den aliphatischen und cycloaliphatischen Kohlenwasserstoffen Cyclohexan und von den sauerstoffhaltigen Kohlenwasserstoffen Propionsäureäthylester und Benzosäureäthylester.

Verwendet werden können auch Lösungsmittelgemische der verschiedenen oben angegebenen organischen Lösungsmittel.

Erfindungsgemäß verwendbare Percarbonsäuren sind Perpropionsäure, Perbuttersäure und Perisobuttersäure. Bevorzugt verwendet werden Perpropionsäure und Perisobuttersäure. Besonders bevorzugt ist Perpropionsäure. Diese Percarbonsäuren, gelöst in einem der genannten organischen Lösungsmittel, können z.B. nach dem in der DE—A—2 262 970 beschriebenen Verfahren hergestellt werden, bei dem wäßriges Wasserstoffperoxid mit der entsprechenden Carbonsäure in Gegenwart von Schwefelsäure umgesetzt und anschließend die entstandene Percarbonsäure mit einem organischen Lösungsmittel aus dem Reaktionsgemisch extrahiert wird, Gegebenenfalls kann die so erhaltene Percarbonsäurelösung in dem organischen Lösungsmittel noch weiter gereinigt werden, insbesondere um den Gehalt an Wasser, Wasserstoffperoxid und Schwefelsäure zu erniedrigen.

Im allgemeinen verwendet man die Percarbonsäuren in Form einer Lösung in einem organischen Lösungsmittel. Derartige Percarbonsäurelösungen können beispielsweise 10 bis 30 Gew.-% der jeweiligen Percarbonsäure, bezogen auf die Lösung, enthalten. Die Allylester können als solche oder ebenfalls gelöst in einem oder mehreren der vorstehend genannten Lösungsmittel eingesetzt werden, wobei beliebig konzentrierte Lösungen der Allylester zum Einsatz gelangen können. Vorzugsweise werden die Allylester als soche eingesetzt und organische Lösungsmittel nur in Form der Percarbonsäurelösung zugegeben.

Das Molverhältnis von eingesetzter Percarbonsäure zu eingesetztem Fumarsäurediallylester kann in weiten Grenzen schwanken. Beispielsweise kann dieses Molverhältnis 0,1:1 bis 10:1 betragen. Bevorzugt wird ein Molverhältnis von 0,15:1 bis 5:1 angewendet. Ganz besonders vorteilhaft ist es, ein Molverhältnis von 0,2 bis 3 Mol Persäure je Mol Fumarsäurediallylester anzuwenden.

Der Wassergehalt der verwendeten Percarbonsäure soll im allgemeinen möglichst niedrig sein. Wassermengen bis 10 Gew.-% in der Percarbonsäurelösung sind im allgemeinen nicht störend. Geeignet ist beispielsweise eine Percarbonsäurelösung mit einem Wassergehält von bis zu 2 Gew.-%. Vorzugsweise verwendet man eine Percarbonsäurelösung, die weniger als 1 Gew.-% Wasser enthält. Besonders bevorzugt ist ein Wassergehalt von weniger als 0,1 Gew.-%.

Der Wasserstoffperoxidgehalt der verwendeten Percarbonsäure in einem der oben genannten organischen Lösungsmittel soll im allgemeinen möglichst niedrig sein. Er kann beispielsweise bis zu 1 Gew.-%, bezogen auf die Percarbonsäurelösung, betragen. Vorteilhaft arbeitet man bei einem Gehalt von weniger als 0,5 Gew.-%. Besonders vorteilhaft ist es, die Umsetzung mit einer Percarbonsäurelösung durchzuführen, die einen Wasserstoffperoxidgehalt unterhalb 0,2 Gew.-% aufweist.

Der Mineralsäuregehalt der verwendeten Percarbonsäure soll möglichst niedrig sein. Vorteilhaft ist es, die Reaktion mit einer Percarbonsäurelösung durchzuführen, die einen Mineralsäuregehalt unterhalb 0,005 Gew.-% besitzt. Besonders vorteilhaft ist ein Mineralsäuregehalt von weniger als 0.001 Gew.-%.

Das erfindungsgemäße Verfahren wird in dem Temperaturbereich von 30—100°C durchgeführt. Bevorzugt arbeitet man bei 40—80°C, besonders bevorzugt bei 50—75°C. In Sonderfällen können die angegebenen Temperaturen auch unter- oder überschritten werden.

Neben der Arbeitsweise unter isothermen Bedingungen, d.h. Einhaltung einer einheitlichen Temperatur in gesamten Reaktionsgemisch, kann man des erfindungsgemäße Verfahren auch unter Ausbildung eines sogenannten Temperaturgradienten durchführen, der im allgemeinen mit fortschreitender Reaktion zunimmit. Man kann aber auch die Reaktion so führen, daß sich mit dem Fortschreiten der Reaktion ein Gradient fallender Temperatur ausbildet.

Das erfindungsgemäße Verfahren kann bei den verschiedensten Drucken durchgeführt werden. Im allgemeinen arbeitet man bei Normaldruck. Das Verfahren kann jedoch auch bei Unter- oder Überdruck durchgeführt werden.

Die Durchführung des erfindungsgemäßen Verfahrens kann diskontinuierlich oder kontinuierlich in den für Umsetzungen dieser Art üblichen Vorrichtungen, wie Rührwerkskessel, Siedereaktoren, Röhrenreaktoren, Schlaufenreaktoren oder Schleifenreaktoren, erfolgen.

Als Werkstoffe für die Reaktionsapparate zur Durchführung des erfindungsgemäßen Verfahrens können beispielsweise Glas, Edelstähle oder emailliertes Material verwendet werden.

Schwermetallionen im Reaktionsgemisch katalysieren die Zersetzung der Percarbonsäure. Man setzt deshalb der Percarbonsäurelösung im allgemeinen Substanzen zu, die Schwermetallionen durch Komplexbildung inaktivieren können. Bekannte Substanzen solcher Art sind beispielsweise Gluconsäure, Äthylendiamintetraessigsäure, Natrium silicat, Natriumpyrophosphat, Natriumhexametaphosphat, Dinatriumdimethylpyrophosphat oder $Na_2$ (2 - Athyl-hexyl)$_5(P_3O_{10})_2$ (vgl. DE—B—1 056 596, Spalte 4, Zeile 60 ff.).

Die Reaktionswärme kann durch innen- oder außenliegende Kühler abgeführt werden. Zur Ableitung der Reaktionswärme kann die Umsetzung auch unter Rückfluß (z.B. in Siedereaktoren) durchgeführt werden.

Der Allylester und die Percarbonsäure können auf beliebige Weise zusammengebracht werden. Beispielsweise kann man beide Komponenten gleichzeitig oder nacheinander in beliebiger Reihenfolge in das Reaktionsgefäß einbringen. Bei diskontinuierlicher Arbeitsweise wird vorzugsweise der Allylester vorgelegt und dann die Percarbonsäurelösung zugegeben. Die Reaktionstemperatur kann dabei vor oder nach der Zugabe der Percarbonsäure eingestellt werden. Man kann auch die beiden Komponenten bei Raumtemperatur gleichzeitig in das Reaktionsgefäß eingeben und dann die Reaktionstemperatur einstellen. Bei kontinuierlicher Arbeitsweise kann man die beiden Komponenten gemeinsam oder getrennt dem Reaktor zuführen. Bei Verwendung mehrerer Reaktoren, die beispielsweise als Kaskade hintereinander geschaltet sein können kann es vorteilhaft sein, den Allylester nur in den ersten Reaktor einzubringen. Man kann die Allylesterzugabe jedoch auch auf mehrere Reaktoren verteilen.

Wenn als Reaktionsprodukt der Fumarsäurediglycidylester angestrebt wird, kann es von Vorteil sein, den Fumarsäurediallylester durch chargenweise Zugabe der Percarbonsäure zu epoxidieren . Dabei kann es von Vorteil sein. Fumarsäureallylglycidylester aus dem Reaktionsgemisch abzutrennen und einer erneuten Umsetzung mit Percarbonsäure zu unterwerfen.

Es kan weiterhin von Vorteil sein, die zur Erreichung des gewünschten Endproduktes Fumarsäureallylglycidylester oder Fumarsäurediglycidylester benötigte Menge Percarbonsäure chargenweise zuzugeben. Bei der chargenweisen Zugabe der Percarbonsäure kann es auch von Vorteil sein, nach Zugabe einzelner Chargen die aus der Percarbonsäure entstandene Carbonsäure aus dem Reaktionsgemisch zu entfernen, z.B. durch Extraktion mit Wasser oder durch Destillation.

Die nach Durchführung der erfindungsgemäßen Verfahrens erhaltenen Reaktionsgemische enthalten im allgemeinen das verwendete organische Lösungsmittel, die aus der Percarbonsäure entstehende Carbonsäure, Fumarsäureallylglycidylester, Fumarsäurediglycidylester und gegebenenfalls nich umgesetzten Fumarsäurediallylester, sowie gegebenenfalls geringe, Mengen von hochsiedenden Nebenprodukten. Im allgemeinen enthält das Reaktionsgemisch aus dem erfindungsgemäßen Verfahren keine Produkte, in denen die zwischen den beiden Carboxylgruppen des Fumarsäurediallylesters befindliche C=C-Doppelbindung epoxidiert worden ist.

Die Aufarbeitung des erhaltenen Reaktionsgemisches erfolgt durch Destillation. Dabei kann man so verfahren, daß man die einzelnen Komponenten in der Reihenfolge ihrer Siedepunkte abdestilliert. Dabei ist es von Vorteil, die Destillation im Vakuum und unter Verwendung von Verdampfern, die kurze Verweilzeiten und eine Verdampfung mit geringer thermischer Belastung des Reaktionsgemisches ermöglichen, durchzuführen. Erfindungsgemäß wird vor der destillativen Aufarbeitung dem Reaktionsgemisch ein geeigneter Zusatzstoff zugefügt, der zwischen der Carbonsäure und dem nächst höher siedenden Bestandteil des Reaktionsgemisches siedet. Dadurch erreicht man eine vollständige Abtrennung der Carbonsäure bei relativ niedrigen Sumpftemperaturen, was hinsichtlich der Minimierung der Bildung von Nebenprodukten aus der Carbonsäure und den epoxidierten Bestandteilen des Reaktionsgemisches von Vorteil ist. Erfindungsgemäß wird dann die Carbonsäure zusammen mit dem Lösungsmittel abdestilliert.

Die weiter Reinigung der gewünschten Endprodukte (Allylglycidylfumarat und/oder Diglycidylfumarat), die nach der oben angeführten Aufarbeitungsmethode erhalten wurden, kann z.B. durch Destillation bei vermindertem Druck erfolgen. Man kann die weitere Reinigung der gewünschten Endprodukte z.B. auch durch Kristallisation aus einem geeigneten Lösungsmittel durchführen. Dadurch ist es möglich, sowohl Allylglycidylfumarat, als auch Diglycidylfumarat in hoher Reinheit zu isolieren.

Es kann vorteilhaft sein, dem Reaktionsgemisch vor oder während der Reaktion und/oder vor oder während der Aufarbeitung Stabilisatoren zuzusetzen, welche die Bildung von Hochsiedern und Polymerisaten verhindern.

Das erfindungsgemäße Verfahren hat eine Reihe von Vorteilen. Mit diesem Verfahren ist es erstmals möglich nach der sogenannten Prileschajew-Reaktion in technischem Maßstab Glycidylester von Fumarsäure herzustellen und dadurch die Nachteile des Epihalogenhydrin-Verfahrens zu vermeiden.

Gegenüber den eingangs zitierten Anwendungen der Pileschajew-Reaktion besteht ein besonderer Vorteil des erfindungsgemäßen Verfahrens darin, daß die aus der Percarbonsäure entstehende Carbonsäure durch solche Maßnahmen aus dem Reaktionsgemisch entfernt werden kann, bei denen keine umweltbelastenden salzhaltigen Abwässer anfallen. Nach dem erfingungsgemäßen Verfahren ist es möglich sowohl Allylglycidylfumarat, als auch Diglycidylfumarat in hoher Reinheit technisch herzustellen.

Das folgende Beispiel erläutert die Erfindung. Sämtliche Prozentangaben sind, soweit nicht anders gesagt, Gewichtsprozente.

Beispiel

196 g (1 Mol) Fumarsäurediallylester wurden bei 70°C mit 675 g einer 20% igen benzolischen Perpropionsäurelösung (1,5 Mol), die einen Wassergehalt von unter 0,1%, einen Wasserstoffperoxidgehalt von unter 0,2% und einen Mineralsäuregehalt von unter 0,001% besaß, umgesetzt. Nach einer Reaktionszeit von 6 Stunden betrug der Persäureumsatz 97%. Nach Abkühlen des Reaktionsgemisches auf Raumtemperatur fügte man 500 ml Benzoesäuremethylester zu und destillierte in einer mit einem Fallstromverdampfer betriebenen Füllkörperkolonne bei einem Druck von 200 mbar Benzol und Propionsäure als gemeinsames Kopfprodukt ab; sie wurden in einer weiteren Kolonne in die reinen Komponenten aufgetrennt.

Das Sumpfprodukt wurde anschließend am Rotationsverdampfer bis zur Trockne eingeengt und aus Benzol umkristallisiert. Fumarsäurediglycidylester wurde dabei in einer Reinheit von 99,2% erhalten.

$$Schmp.=78—80°C$$

Die Elementaranalyse ergab folgende Werte:

|     | berechnet | gefunden |
| --- | --------- | -------- |
| C:  | 52,6%     | 52,5%    |
| H:  | 5,3%      | 5,3%     |
| O:  | 42,0%     | 41,8%    |

Die benzolische Mutterlauge, die nichtumgesetzten Fumarsäurediallylester und Fumarsäureallylglycidylester enthielt, wurde zur erneuten Umsetzung in den Reaktor zurückgeführt.

**Patentansprüche**

1. Verfahren zur Herstellung von Fumarsäureglycidylestern, dadurch gekennzeichnet, daß man Fumarsäurediallylester mit einer 3 bis 4 Kohlenstoffatome enthaltenden Percarbonsäure in Gegenwart von organischen Lösungsmitteln bei einer Temperatur von 30 bis 100°C umsetzt und anschließend das erhaltene Reaktionsgemisch durch Destillation aufarbeitet, wobei man die aus der Percarbonsäure entstandene Carbonsäure abtrennt, indem man dem Reaktionsgemisch einen Zusatzstoff zufügt, der zwischen der aus der Percarbonsäure entstehenden Carbonsäure und dem nächst höher siedenden Bestandteil des Reaktionsgemisches siedet und die Carbonsäure zusammen mit dem Lösungsmittel abdestilliert.

2. Verfahren nach Anspruch 1, dadurch gekennzeichnet, daß man Perpropionsäure einsetzt.

3. Verfahren nach Anspruch 1, und 2, dadurch gekennzeichnet, daß man dem Umsetzung in Gegenwart von 1,2 - Dichlorpropan, Tetrachlorkohlenstoff, Benzol, Chlorbenzol, Cyclohexan und/oder Propionsäureäthylester durchführt.

4. Verfahren nach Anspruch 1 bis 3, dadurch gekennzeichnet, daß man percarbonsäure in Form einer Lösung in einem organischen Lösungsmittel verwendet, wobei die Lösung weniger als 10 Gew.-% Wasser, weniger als 1 Gew.-% Wasserstoffperoxid und weniger als 0,005 Gew.-% Mineralsäure enthält.

5. Verfahren nach Anspruch 1 bis 4, dadurch gekennzeichnet, daß man 0,1 bis 10 Mol Percarbonsäure pro Mol Allylester anwendet.

6. Verfahren nach Anspruch 1 bis 5, dadurch gekennzeichnet, daß man dem Reaktionsgemisch vor oder während der Reaktion und/oder vor oder während der Aufarbeitung Stabilisatoren zusetzt, welche die Bildung von Hochsiedern und Polymerisaten verhindern.

7. Verfahren nach Anspruch 1 bis 6, dadurch gekennzeichnet, daß man die gewünschten Endprodukte durch Destillation oder Kristallisation weiter reinigt.

**Revendications**

1. Procédé de production d'esters de glycidyle de l'acide fumarique, caractérisé en ce qu'on fait réagir l'ester diallylique de l'acide fumarique avec un acide percarboxylique contenant 3 à 4 atomes de carbone en présence de solvants organiques à une température de 30 à 100°C, puis on traite par distillation le mélange réactionnel obtenu, et on sépare alors l'acide carboxylique produit à partir de l'acide percarboxylique en ajoutant au mélange réactionnel un additif qui bout entre l'acide carboxylique produit à partir de l'acide percarboxylique et le composant du mélange réactionnel de point d'ébullition immédiatement supérieur et on distille l'acide carboxylique conjointement avec le solvant.

2. Procédé suivant la revendication 1, caractérisé en ce qu'on utilise l'acide perpropionique.

3. Procédé suivant les revendications 1 et 2, caractérisé en ce qu'on conduit la réaction en présence de 1,2-dichloropropane de tétrachlorure de carbone, de benzène, de chlorobenzène, de cyclohexane et/ou d'ester éthylique d'acide propionique.

4. Procédé suivant les revendications 1 à 3, caractérisé en ce qu'on utilise l'acide percarboxylique sous la forme d'une solution dans un solvant organique cette solution contenant moins de 10% en poids d'eau, moins de 1% en poids de peroxyde d'hydrogène et moins de 0,005% en poids d'acide minéral.

5. Procédé suivant les revendications 1 à 4, caractérisé en ce qu'on utilise 0,1 à 10 moles d'acide percarboxylique par mole d'ester allylique.

6. Procédé suivant les revendications 1 à 5, caractérisé en ce qu'on ajoute au mélange réactionnel avant ou pendant la réaction et/ou avant ou pendant le traitement des agents stabilisants qui empêchent la formation de corps de haut point d'ébullition et de produits de polymérisation.

7. Procédé suivant les revendications 1 à 6, caractérisé en ce qu'on purifie encore les produits finals désirés par distillation ou par cristallisation.

**Claims**

1. Process for the preparation of fumaric acid glycidyl esters, characterised in that fumaric acid diallyl ester is reacted with a percarboxylic acid containing 3 to 4 carbon atoms in the presence of organic solvents at a temperature of 30 to 100°C and then the reaction mixture obtained is worked up by distillation, the carboxylic acid formed from the percarboxylic acid being separated off, by adding to the reaction mixture an additive which has a boiling point between that of the carboxylic acid forming from the percarboxylic acid and that of the constituent of the reaction mixture with the next highest boiling point and the carboxylic acid is distilled off together with the solvent.

2. Process according to Claim 1, characterised in that perpropionic acid is employed.

3. Process according to Claim 1 and 2, characterised in that the reaction is carried out in the presence of 1,2-dichloropropane, carbon tetrachloride, benzene, chlorobenzene, cyclohexane and/or ethyl propionate.

4. Process according to Claim 1 to 3, characterised in that the percarboxylic acid is used in the form of a solution in an organic solvent, the solution containing less than 10% by weight of water, less than 1% by weight of hydrogen peroxide and less than 0.005% by weight of mineral acid.

5. Process according to Claim 1 to 4, characterised in that 0.1 to 10 mols of percarboxylic acid are used per mol of allyl ester.

6. Process according to Claim 1 to 5, characterised in that stabilisers which prevent the formation of high-boiling constituents and polymers are added to the reaction mixture before or

11

0 008 115

12

during the reaction and/or before or during the working up.

7. Process according to Claim 1 to 6, characterised in that the desired end products are further purified by distillation or crystallisation.